# DEMANDE DE BREVET EUROPEEN

(11) **EP 0 719 528 A2**
(43) Date de publication de la demande: **03.07.1996**
(21) Numéro de dépôt: 95402959.1
(22) Date de dépôt: 28.12.1995
(51) Int. Cl.: A61F 2/26

(54) **Implants péniens extra caverneux**

(30) Priorité: 28.12.1994 FR 9415763
(71) Demandeur: Subrini, Louis, Dr., F-78370 Plaisir (FR)
(72) Inventeur: Subrini, Louis, Dr., F-78370 Plaisir (FR)

(57) **Abrégé**

L'invention concerne un implant pénien extra-caverneux permettant d'augmenter le volume de la verge.

Il se compose de deux joues latérales indépendantes 1 dont la section transversale est en forme de croissant, dont la face externe 2 est convexe et la face interne 3 est concave ou plane. Elles recouvrent les faces latérales des corps caverneux, sans recouvrir leurs faces supérieure et inférieure.
Chaque joue présente une diminution progressive de son épaisseur au niveau de son extrémité distale dont le bord libre est situé dans un plan oblique taillé aux dépens du bord supérieur, pour s'adapter à l'anatomie du sillon balanique au fond duquel il s'appuie.

Le dispositif selon l'invention est particulièrement destiné à augmenter le diamètre et la longueur de la verge.

## Description

La présente invention concerne un implant pénien extra-caverneux destiné à augmenter le volume de la verge.

Les techniques actuelles d'augmentation de volume de la verge, font appel à la mise en place de graise sous la peau du fourreau, qui présentent de multiples inconvénients :
1. La graisse a une forte tendance à l'inflammation, ce qui conduit souvent au rejet, ou oblige à l'ablation.
2. La résorption au moins partielle de la graisse entraîne une forte diminution de son volume initial.
3. La longueur de la verge n'est pas significativement augmentée.
On pourrait aussi pour augmenter le volume de la verge, insérer une prothèse pénienne quelconque. L'inconvénient en pareil cas est que les prothèses sont mises en place dans le corps caverneux qui est l'organe de l'érection, avec le risque évident d'entraîner une impuissance.

On pourrait également envisager l'insertion sous la peau d'une prothèse impaire et médiane. En pareil cas elle ne pourrait être que d'une section faible, n'entraînant pas d'augmentation significative de volume, sauf à déformer la verge qui serait alors plus haute que large, ce qui est à l'inverse de l'anatomie normale. Un autre inconvénient est que la zone d'appui distal de la prothèse au niveau du gland, ne se ferait qu'au niveau de la face dorsale sans aucun soutien latéral avec le risque soit de déformation du gland, soit de perforation du fait de l'excès de pression. D'une façon générale, les prothèses péniennes connues sont conçues pour permettre la pénétration à des patients impuissants, et non pour augmenter le volume de la verge chez des individus à érection normale.

La présente invention se propose de remédier aux inconvénients et aux insuffisances des techniques ci-dessus.

Le but est d'augmenter le volume global de la verge, c'est à dire son diamètre et sa longueur, grâce à un implant pénien qui recouvre une partie des corps caverneux, dans leur segment pénien, ou "penis pendulum".

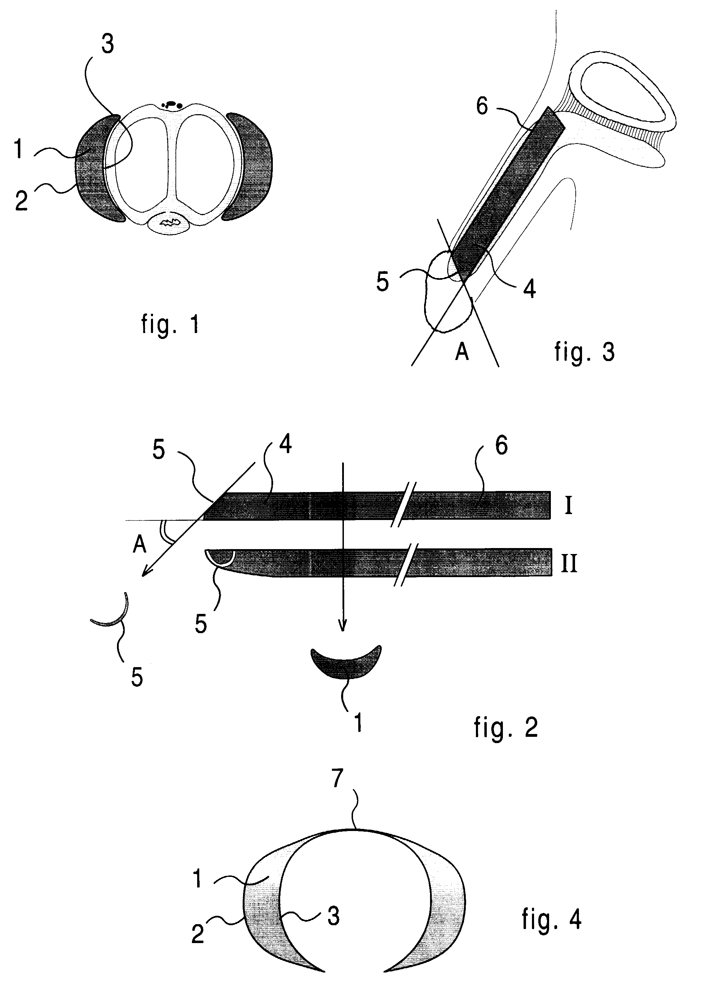

L'implant pénien selon une première caractéristique, est composé de deux joues indépendantes l'une de l'autre, qui recouvrent les faces latérales des corps caverneux, sans recouvrir leurs faces supérieure et inférieure. Le section transversale de chacune des deux joues est de préférence en forme de croissant, comme illustré dans la figure 1.

Selon des modalités particulières de réalisation,
- le bord distal peut être situé dans un plan oblique par rapport au grand axe de l'implant.
- le dispositif peut présenter des perforations.
- les deux joues peuvent être réunies l'une à l'autre par un feuille mince.
- le dispositif peut présenter une ou plusieurs zone formée d'un matériau secondairement réhabitable par les tissus voisins.

Les dessins annexés illustrent l'invention.

La figure 1 représente une coupe du dispositif selon l'invention, mis en place.

La figure 2 représente une vue latérale de la joue gauche : en vue gauche I, et en vue supérieure II, ainsi que deux coupes en des points différents.

La figure 3 représente une vue latérale gauche de la joue gauche, en place dans la verge.

La figure 4 représente en coupe transversale une variante du dispositif, où les joues latérales sont réunies par une feuille mince.

En référence à ces dessins, l'implant pénien selon une première caractéristique, se présente sous la forme de deux corps allongés composés de deux joues ou segments, l'un gauche et l'autre droit, indépendants l'un de l'autre, dont la section transversale est en forme de croissant. La face externe 2 est convexe, et la face interne 3 est concave tel que représenté dans la figure 1. La concavité peut être plus ou moins prononcée, et même être nulle, la face interne devenant plane, la joue ayant alors la forme d'un hemi-cylindre, non représenté. Les deux joues sont disposées de part et d'autre des corps caverneux, de telle sorte qu'ils embrassent ses faces latérales, sans recouvrir les faces supérieure et inférieure.

Les bords peuvent être laissés libres, ou être cousus au corps caverneux, soit directement, soit par l'intermédiaire d'une bandelette de renforcement.

Chaque joue présente une extrémité distale et une extrémité proximale. L'extrémité distale 4 peut présenter une diminution progressive de son épaisseur comme illustré par la figure 2. Le bord libre 5 de l'extrémité distale peut être taillé en oblique aux dépens de son bord supérieur, selon un angle A qui est de l'ordre de 45° environ, de manière à se conformer à l'anatomie du sillon balanique sur lequel elle vient prendre appui, comme représenté sur les figures 2 et 3. L'extrémité proximale 6 prend un appui opposé au niveau du ligament suspenseur de la verge. Cette extrémité 6 est destinée à être coupée sur mesure à la longueur désirée par le chirurgien. Si la mesure est prise sur la verge en extension, le dispositif permet de maintenir une augmentation de la longueur de la verge au repos, qui vient s'ajouter à l'augmentation de diamètre pour réaliser une importante augmentation du volume global de l'ensemble. Le dispositif est constitué d'un matériau synthétique bio-compatible. Il peut être soit à l'état solide, et alors d'une dureté comprise entre 25 et 50 shore A environ, soit être à l'état de gel ou analogue. Dans ce dernier cas, il est entouré d'une enveloppe appropriée.

Selon une des modalités de l'invention, le dispositif peut présenter une ou plusieurs zones formées d'un matériau réhabitable tel qu'un treillis de polyester par exemple. La réhabitation ultérieure par les tissus voisins s'opposant à tout déplacement secondaire de l'implant. La bandelette elle-même (non représentée) peut être constituée d'un tel matériau réhabitable.

Selon une autre modalité de l'invention, les deux joues latérales peuvent être réunies par une feuille 7 beaucoup plus mince que les segments latéraux 1 comme illustré par la figure 4.

Selon une autre modalité de l'invention, l'ensemble du dispositif peut être plus fermé, et même complètement fermé (non illustré) réalisant alors un cylindre creux.

Pour éviter la multiplication des figures, on n'a représenté dans la planche des figures que des exemples de réalisation, mais les divers segments du dispositif ou les bandelettes, pourront être de situation, de forme ou d'épaisseur variable selon les besoins. C'est ainsi que selon une autre modalité de l'invention, le dispositif peut présenter des perforations ou des cannelures non représe tées, afin de donner plus de légèreté ou de souplesse à l'ensemble.

Les indications principales sont représentées par les pathologies d'atrophie pénienne, congénitales ou acquises, à érection normale. Le dispositif permet un allongement et une augmentation de diamètre de la verge au repos, sans pour autant empêcher l'érection physiologique, puisque les implants ne recouvrent qu'une faible partie des corps caverneux et n'empêchent donc pas l'augmentation de circonférence de l'érection physiologique.

Enfin au cas de déficit modéré de l'érection sans impuissance vraie, le choix d'un matériau de dureté comprise entre 25 et 50 shore A, assure le complément de rigidité pour permettre une pénétration dans des conditions satisfaisantes.

## Revendications

1. Implant pénien extra-caverneux pour augmenter le volume global de la verge, par augmentation conjointe de la longueur et du diamètre. Caractérisé en ce qu'il est composé de deux joues indépendantes l'une de l'autre chacune étant en coupe transversale en forme de croissant, de façon à recouvrir les faces latérales des corps caverneux en laissant libres leurs faces supérieure et inférieure.

2. Implant pénien selon la revendication précédente caractérisé en ce que l'extrémité d'appui distale de chaque joue est située dans un plan oblique taillé aux dépens du bord supérieur.

3. Implant pénien selon l'une quelconque des revendications précédentes, caractérisé en ce que l'implant est constitué d'une matière synthétique d'une dureté comprise entre environ 25 et 50 shore A.

4. Implant pénien selon l'une quelconque des revendications précédentes, caractérisé en ce qu'il présente des perforations.

5. Implant pénien selon l'une des revendications précédentes caractérisé en ce que les deux segments latéraux sont réunis l'un à l'autre par une feuille mince.

6. Implant pénien selon l'une des revendications précédentes, caractérisé en ce qu'il comporte une ou plusieurs bandelettes de renforcement.

7. Implant pénien selon l'une quelconque des revendications précédentes, caractérisé en ce qu'il présente une ou plusieurs zones formées d'un matériau secondairement réhabitable par les tissus voisins.
